# EUROPEAN PATENT SPECIFICATION

(11) **EP 3 086 798 B1**
(45) Date of publication and mention of the grant of the patent: **07.11.2018**
(21) Application number: 14833247.1
(22) Date of filing: 19.12.2014
(51) Int. Cl.: A61K 31/4045, A61K 31/7048, A61K 36/481, A61K 45/06, A61P 25/00, A61K 47/69

(54) **FORMULATIONS BASED ON ASTRAGALOSIDE IV OR EXTRACTS CONTAINING IT FOR THE PREVENTION AND TREATMENT OF INSOMNIA AND JET-LAG-RELATED DISORDERS**
FORMULIERUNGEN AUF BASIS VON ASTRAGALOSID IV ODER EXTRAKTEN DAMIT ZUR VORBEUGUNG UND BEHANDLUNG VON DURCH SCHLAFLOSIGKEIT UND JET-LAG VERMITTELTEN ERKRANKUNGEN
FORMULATIONS À BASE D'ASTRAGALOSIDE IV OU D'EXTRAITS EN CONTENANT POUR LA PRÉVENTION ET LE TRAITEMENT DE L'INSOMNIE ET DES TROUBLES LIÉS AU DÉCALAGE HORAIRE

(30) Priority: 23.12.2013 IT MI20132194
(43) Date of publication of application: 02.11.2016
(73) Proprietor: Velleja Research SRL, 29010 Pontenure (PC) (IT)
(72) Inventor: DI PIERRO, Francesco, I-29010 Pontenure (PC) (IT)
(74) Representative: Bianchetti Bracco Minoja S.r.l.
(86) International application number: PCT/IB2014/067127
(87) International publication number: WO 2015/097611

(56) References cited:
- CN-A- 101 062 159
- CN-C- 1 171 591
- JP-A- 2008 174 512
- DATABASE WPI Week 201369 Thomson Scientific, London, GB; AN 2013-Q21667 XP002720841, & CN 103 071 048 A (GUIZHOU SHENQI INVESTMENT CO LTD) 1 May 2013 (2013-05-01)

## Description

The present invention relates to formulations based on astragaloside IV or extracts containing it for the prevention and treatment of insomnia and jet-lag-related disorders.

### Prior art

Jet lag is a syndrome involving numerous variables (biological, environmental and climatic). It is mainly caused by a lack of synchronisation between the circadian rhythms to which the body is used, and the new cycles of light and darkness typical of the destination.

Disorganisation of the internal biological clock (which also regulates the sleep-waking cycle) as a result of new light/dark periods gives rise to a series of disorders collectively called "jet-lag syndrome". Said disorders include daytime asthenia (tiredness and fatigue during the day), loss of appetite, general malaise, nausea, headache, aching muscles, sleep disorders associated with a melatonin secretion imbalance, excessive drowsiness, insomnia, and difficulty falling asleep; reduced physical and mental performance, difficulty in concentrating or performing normal activities, low or altered mood, irritability, agitation, alterations in gastrointestinal functionality, digestive problems, stomach disorders, constipation or diarrhoea.

*Astragalus membranaceus* is an oriental plant which is highly prized in both traditional and allopathic medicine. The root of this species, which is rich in high-molecular-weight polysaccharides, possesses tonic, adaptogenic and immunomodulating properties.

In addition to the activities described above, astragalus extract has also been proposed for preventing or combating insomnia and/or sleep quality disorders.

CN 103071048 discloses a chinese medicine composition comprising 5-30 pts. wt. astragalus root and 2-30 pts. wt. melatonin in the form of a tablet.

CN 1 171 591 discloses capsules comprising melatonin and astragalus root extracts for improving sleep and JP 2008 174512 discloses the use of a composition for improving sleep or treat sleeplessness comprising DHA and vitamin E.

CN 101 062 159 discloses a water-alcohol Astragalus extract.

### Description of the invention

It has now been discovered that astragaloside IV boosts the sedative effect of melatonin to a surprising extent. The synergic effect is particularly evident when the weight ratio between astragaloside and melatonin is between 20:1 and 5:1.

The present invention therefore relates to compositions containing melatonin and astragaloside IV or the equivalent in extracts containing it in *Astragalus membranaceus* extracts, typically aqueous or water-alcohol extracts.

A 20:1 weight ratio between astragaloside IV and melatonin is preferred.

The unit doses of melatonin range from 0.5 to 5 mg, preferably from 0.5 to 3 mg, and more preferably from 0.5 to 1 mg.

The doses of astragaloside IV (or the equivalent in extracts containing it) range from 2.5 mg to 100 mg, preferably from 5 to 75 mg, and more preferably from 5 to 20 mg.

According to a preferred aspect of the invention, astragaloside IV can be in free form or phospholipid-complexed, in particular with soya distearoyl-phosphatidylcholine or phosphatidylserine, in terclathrate form or co-ground with beta-cyclodextrins. Similarly, the extract containing astragaloside, preferably a dried extract, may be in free form, phospholipid-complexed, in terclathrate form or co-ground with beta-cyclodextrins. Suitable carriers include oils, lipids and/or amino acids.

The formulations according to the invention can also contain other ingredients able to exercise a psychotropic, sedative, tranquillising and anti-anxiety action such as St. John's Wort, kawakawa, passion fruit, chamomile, valerian, lemon balm and linden extracts, and ingredients with an antioxidant action (which therefore stabilise the formula) can be added, such as polyphenols, vitamins A, C and E, ubidecarenone, glutathione, selenium, lipoic acid.

The compositions according to the invention can be formulated in a way suitable for oral administration, and will be prepared by conventional methods well known in pharmaceutical technology, such as those described in "Remington's Pharmaceutical Handbook", Mack Publishing Co., N.Y., USA, using excipients, diluents, fillers, anti-caking agents, flavourings and sweeteners acceptable for their final use.

In particular, the compositions according to the invention can be formulated as conventional, film-coated, multilayer, differentiated-release or gastroprotected tablets, soft or hard gelatin capsules, granulates, sachets, suspensible or effervescent powder, medicated gummy lozenges, effervescent tablets or orodispersible powder.

### Pharmacological tests

The effect of the association according to the invention has been demonstrated in a sedation model developed in the Sprague-Dawley rat (Simonsen Laboratories Inc., Gilford, CA), wherein the kinetic activity of the animals was monitored for 24 hours with a photoelectric cell that counts the movements of the animals in the cage.

It was observed that the sedative action of melatonin only appears if an astragalus extract containing astragaloside IV combined with melatonin is administered to the animal. In fact, neither the administration of astragalus extract only, with or without astragaloside, nor the administration of astragalus extract with astragaloside but without melatonin, produces a sedative effect compared with the control group. Conversely, the administration of astragalus extracts with astragaloside or of pure astragaloside combined with melatonin produces a highly significant sedative effect. This phenomenon appears at melatonin doses described as totally ineffective in terms of sedation in the rat. To obtain the same sedation with melatonin only, a dose of melatonin 20 times higher would have to be administered to the animal (see table 1).

**Table 1. Effect of various pharmacological treatments, measured cumulatively by photocell in the 24 hours after treatment, on the spontaneous locomotor activity of SD rats (N=8/cage).**

| **Treatment** | **no. of counts/24 hours** | **p** |
|---|---|---|
| Control | 8037 | |
| Astragalus extract 0% astragaloside IV (os) 100 mg/rat | 8122 | |
| Astragalus extract 20% astragaloside IV (os) 100 mg/rat | 8097 | |
| Astragaloside IV (os) 20 mg/rat | 7986 | |
| Melatonin 2.0 mg/kg (os) | 8210 | |
| Melatonin 40 mg/kg (os) | 4354 | <0.01 |
| Astragalus extract 0% astragaloside IV (os) 100 mg/rat + | | |
| Melatonin 2.0 mg/kg (os) | 8189 | |
| Astragalus extract 20% astragaloside IV (os) 100 mg/rat + | | |
| Melatonin 2.0 mg/kg (os) | 4523 | <0.01 |
| Astragaloside IV (os) 20 mg/rat + | | |
| Melatonin 2.0 mg/kg (os) | 4366 | <0.01 |

Some examples of formulations are set out below.
EXAMPLE 1: FILM-COATED TABLETS

| | |
|---|---|
| Melatonin: | 1 mg/dose |
| Astragalus extract (10% astragaloside IV): | 200 mg/dose |

EXAMPLE 2: THREE-LAYER TABLETS
1 st fast-release layer

| | |
|---|---|
| Astragalus extract (titrated to 20%): | 100 mg/dose |
| Melatonin: | 1 mg/dose |

2nd normal-release layer

| | |
|---|---|
| Astragalus extract (titrated to 20%): | 100 mg/dose |
| Melatonin: | 1 mg/dose |

3rd slow-release layer

| | |
|---|---|
| Salvia miltiorrhiza extract: | 25 mg/dose |
| Melatonin: | 1 mg/dose |

EXAMPLE 3: GASTROPROTECTED TABLETS

| | |
|---|---|
| Astragaloside IV: | 20 mg/dose |
| Melatonin: | 1 mg/dose |

EXAMPLE 4: OROSOLUBLE SACHETS

| | |
|---|---|
| Astragaloside IV: | 20 mg/dose |
| Melatonin: | 1 mg/dose |

EXAMPLE 5: SOFT GELATIN CAPSULES

| | |
|---|---|
| Astragaloside IV: | 10 mg/dose |
| Melatonin: | 0.5 mg/dose |

## Claims

1. Compositions comprising melatonin in unit doses ranging from 0.5 to 5 mg, and astragaloside IV or the equivalent in Astragalus extracts containing it, in unit doses ranging from 2.5 to 100 mg, the weight ratio between astragaloside and melatonin ranging between 20:1 and 5:1.

2. Compositions according to claim 1 wherein the Astragalus extracts are aqueous or water-alcohol extracts.

3. Compositions according to the above claims wherein astragaloside IV or the Astragalus extract containing it is present as a dried extract, free or complexed with phospholipids, in a terclathrate form or in a form co-ground with beta-cyclodextrins.

4. Compositions according to the above claims further comprising extracts of St. John's Wort, kawakawa, passion fruit, chamomile, valerian, lemon balm and linden.

5. Compositions according to the above claims further comprising ingredients with antioxidant action selected from polyphenols, vitamins A, C, E, ubidecarenone, glutathione, selenium, lipoic acid.

6. Compositions according to the above claims in the form of conventional tablets, filmed tablets, multilayer tablets, differentiated-release tablets, gastro-resistant tablets, soft- or hard-gelatin capsules, granulates, sachets, suspensible or effervescent powder, gummy lozenges, effervescent tablets or orodispersible powder.

7. Compositions according to the above claims for use in the treatment of insomnia and jet-lag disorders.

## Patentansprüche

1. Zusammensetzungen umfassend Melatonin in Einheitsdosen im Bereich von 0,5 bis 5 mg und Astragalosid IV oder das Äquivalent in Form von Astragalusextrakten, die es enthalten, in Einheitsdosen im Bereich von 2,5 bis 100 mg, wobei das Gewichtsverhältnis zwischen Astragalosid und Melatonin im Bereich zwischen 20:1 und 5:1 liegt.

2. Zusammensetzungen nach Anspruch 1, wobei die Astragalusextrakte wässrige Extrakte oder Wasser-Alkoholextrakte sind.

3. Zusammensetzungen nach den obigen Ansprüchen, wobei Astragalosid IV oder das Astragalusextrakt, das es enthält, als getrocknetes Extrakt frei von oder komplexiert mit Phospholipiden, in Terclathratform oder in einer mit Beta-Cyclodextrin zusammengemahlenen Form vorliegt.

4. Zusammensetzungen nach den obigen Ansprüchen, ferner Extrakte von Johanniskraut, Kawakawa, Passionsfrucht, Kamille, Baldrian, Zitronenmelisse und Linde umfassend.

5. Zusammensetzungen nach den obigen Ansprüchen, ferner Bestandteile mit Antioxidationswirkung ausgewählt unter Polyphenolen, Vitaminen A, C, E, Ubidecarenon, Glutathion, Selenium, Liponsäure umfassend.

6. Zusammensetzungen nach den obigen Ansprüchen in Form herkömmlicher Tabletten, mit Film überzogener Tabletten, Mehrschichttabletten, verschiedentlich freigesetzter Tabletten, magensaftresistenter Tabletten, Weich- oder Hartgelatinekapseln, Granulaten, Beutelchen, suspendierbarer oder brausender Pulver, Gummibonbons, Brausetabletten oder Schmelztabletten.

7. Zusammensetzungen nach den obigen Ansprüchen zur Verwendung bei der Behandlung von Schlaflosigkeit und Jetlagbeschwerden.

## Revendications

1. Compositions comprenant de la mélatonine en doses unitaires se situant dans la plage allant de 0,5 à 5 mg et de l'astragaloside IV ou l'équivalent dans des extraits d'Astragalus le contenant, en doses unitaires se situant dans la plage allant de 2,5 à 100 mg, le rapport en poids entre l'astragaloside et la mélatonine se situant dans la plage entre 20 : 1 et 5 : 1.

2. Compositions selon la revendication 1 dans lesquelles les extraits d'Astralagus sont des extraits aqueux ou d'eau-alcool.

3. Compositions selon les revendications ci-dessus dans lesquelles l'astragaloside IV ou l'extrait d'Astragalus le contenant est présent sous forme d'extrait séché, exempt ou complexé avec des phospholipides, sous forme de terclathrate ou sous forme co-hachée avec des béta-cyclodextrines.

4. Compositions selon les revendications ci-dessus comprenant en outre des extraits de St John Wort, de kawakawa, de fruit de la passion, de camomille, de valériane, de baume de citron et de tilleul.

5. Compositions selon les revendications ci-dessus comprenant en outre des ingrédients avec une action antioxydante choisis parmi les polyphénols, les vitamines A, C, E, l'ubidécarénone, le glutathion, le sélénium, l'acide lipoïque.

6. Compositions selon les revendications ci-dessus sous forme de comprimés classiques, de comprimés filmés, de comprimés multi-couches, de comprimés à libération différentiée, de comprimés gastro-résistants, de capsules de gélatine molle ou dure, de granulés, de sachets, de poudre suspensible ou effervescente, de pastilles gommeuses, de comprimés effervescents ou de poudre orodispersible.

7. Compositions selon les revendications ci-dessus destinées à être utilisées dans le traitement de l'insomnie et des troubles du décalage horaire.
